**Europäisches Patentamt**

**European Patent Office**

**Office européen des brevets**

(19)

(11) Veröffentlichungsnummer: **0 143 245**
**B1**

(12) # EUROPÄISCHE PATENTSCHRIFT

(45) Veröffentlichungstag der Patentschrift:
24.02.88

(21) Anmeldenummer: 84111267.5

(22) Anmeldetag: 21.09.84

(51) Int. Cl.⁴: **C 07 C 69/30,** C 07 C 69/26,
C 07 C 67/26, A 61 K 7/48,
B 01 F 17/34

(54) **Neue O/W-Emulgatoren für kosmetische Zwecke.**

(30) Priorität: 27.10.83 DE 3338890

(43) Veröffentlichungstag der Anmeldung:
05.06.85 Patentblatt 85/23

(45) Bekanntmachung des Hinweises auf die Patenterteilung:
24.02.88 Patentblatt 88/8

(84) Benannte Vertragsstaaten:
AT BE CH DE FR GB IT LI NL SE

(56) Entgegenhaltungen:
AT-B-263 975
DE-A-2 024 051
DE-B-2 023 786
GB-A-891 901

(73) Patentinhaber: Beiersdorf Aktiengesellschaft,
Unnastrasse 48, D-2000 Hamburg 20 (DE)
Patentinhaber: HOECHST AKTIENGESELLSCHAFT,
Postfach 80 03 20, D-6230 Frankfurt am Main 80
(DE)

(72) Erfinder: Hoppe, Udo, Dipl.- Chem. Dr., Lottbeker
Weg 7, D-2000 Hamburg 65 (DE)
Erfinder: Quack, Jochen M. Dipl.- Chem. Dr.,
Wilhelm- Reuter- Strasse 16, D-6239
Eppstein/Taunus (DE)
Erfinder: Reng, Alwin, Dipl.- Ing., Im
Schulzehnten 22, D-6233 Kelkheim/Taunus (DE)
Erfinder: Stühler, Herbert, Dipl.- Chem. Dr.,
Hochfellnstrasse 12, D-8269 Burgkirchen/Alz (DE)
Erfinder: Wittern, Klaus- Peter, Dipl.- Phys. Dr.,
Wurmkamp 19, D-2000 Schenefeld (DE)

**0 143 245**

**Beschreibung**

Die Erfindung betrifft neue Öl-in-Wasser-(O/W)-Emulgatoren, ein Verfahren zu ihrer Herstellung und sie enthaltende kosmetische Zubereitungen.

Es ist bekannt, daß kosmetische Zubereitungen, die Wollwachs oder Wollwachs-Derivate enthalten, bei ihrer Anwendung auf der Haut leicht in diese eindringen, eine hauterweichende und geschmeidigmachende Wirkung aufweisen und somit wertvolle hautpflegende Mittel darstellen. Dies ist im wesentlichen darauf zurückzuführen, daß die Grundsubstanz, das Wollwachs (Lanolin), das aus Schafswolle gewonnen wird, in seiner chemischen Zusammensetzung sowie in seinen physiologischen Eigenschaften von allen natürlichen Grundstoffen dem menschlichen Haut- und Haarfett am nächsten kommt (J.S. Jellinek, "Kosmetologie", 2. Aufl., Dr. Alfred Hüthig Verlag, Heidelberg (1967), S. 151).

Unter Verwendung von durch Verseifung von Wollwachs gewonnenen Wollwachsalkoholen hergestellte W/O-Emulsionen stellen bedingt durch die hervorragenden Emulgatoreigenschaften der Wollwachsalkohole Produkte mit ausgezeichneten Eigenschaften dar, die in Form von Cremes, Salben und flüssigen Emulsionen eine besondere gute Beständigkeit aufweisen und hierdurch bedingt eine weite Verbreitung gefunden haben.

Auch mit den in der DE-PS-2 023 786 beschriebenen Estern aus Wollwachssäuren und Glycerin, insbesondere den Partialestern, die in ihrem Emulgiervermögen den Wollwachsalkoholen zumindest gleichwertig, zumeist jedoch überlegen sind, lassen sich W/O-Emulsionen herstellen, die sich neben einer guten Beständigkeit durch einen besonders schönen Glanz auszeichnen. Diese Ester haben gegenüber anderen Emulgatoren auf Wollwachsbasis den großen Vorteil, daß sie aus den bei der Verseifung des Wollwachses zur Gewinnung der Wollwachsalkohole in erheblicher Menge als unerwünschtes Nebenprodukt anfallenden Wollwachssäuren, für die man bisher trotz großer Bemühungen keine rechte Verwendung gefunden hat, durch Veresterung mit Glycerin in einfacher Weise preisgünstig gewonnen werden können. Glycerin stellt bekanntlich ebenfalls ein Nebenprodukt dar, das bei der Verseifung von pflanzlichen und tierischen Fetten im Zuge der Seifenherstellung in größeren Mengen anfällt.

Sowohl die Wollwachsalkohole als auch die Wollwachssäuren-Glycerin-Ester sind ausgeprägte W/O-Emulgatoren, die in Mineralölen, Isopropylmyristat, Ricinusöl und ähnlichen ölartigen Substanzen gut löslich, dagegen in Wasser unlöslich sind.

Da zur Herstellung spezieller kosmetischer Präparate zumindest eine beschränkte Wasserlöslichkeit des Emulgators erwünscht ist, bestand die Aufgabe der Erfindung darin, Verbindungen aufzufinden und Verfahren zu ihrer Herstellung zu schaffen, die unter Beibehaltung der besonders vorteilhaften Eigenschaften von Wollwachs-Derivaten enthaltenden Zubereitungen in Abänderung ihrer grenzflächenaktiven Eigenschaften eine ausreichende Wasserlöslichkeit zeigen.

Eine weitere Aufgabe der Erfindung bestand darin, den auf einfache und preisgünstige Weise erhältlichen Wollwachssäuren-Glycerin-Estern, insbesondere den Partialestern des Glycerins mit Wollwachssäuren, neue Anwendungsbereiche zu erschließen.

Es wurde gefunden und hierin liegt die Lösung dieser Aufgaben, daß ausgehend von den Estern der Wollwachssäuren mit Glycerin, insbesondere den Partialestern, durch Polyethoxylierung (Umsetzung mit Ethylenoxid) Produkte erhalten werden können, die eine gute Wasserlöslichkeit zeigen und darüber hinaus ausgeprägte O/W-Emulgatoren darstellen. Dabei kann bei der Umsetzung das Molverhältnis zwischen den Wollwachssäuren-Glycerin-Estern und Ethylenoxid je nach den angestrebten Eigenschaften der Umsetzungsprodukte in weiten Grenzen variiert werden.

Gegenstand der Erfindung sind daher neue O/W-Emulgatoren für kosmetische Zwecke in Form von polyethoxylierten Wollwachs-Derivaten, die durch Umsetzung der Voll- oder Partialester aus Wollwachssäuren und Glycerin mit Ethylenoxid erhältlich sind, wobei das Molverhältnis von Ester zu Ethylenoxid 1 : (0,5 bis 30) beträgt.

Unter der Bezeichnung "Partialester" sollen solche Ester der Wollwachssäuren mit Glycerin verstanden werden, bei denen nur ein Teil, vorzugsweise etwa 40 bis 60 %, der ursprünglichen freien Hydroxylgruppen des Glycerins mit den Wollwachssäuren verestert sind, die somit noch freie OH-Gruppen enthalten. Diese Partialester weisen beispielsweise OH-Zahlen von etwa 120 bis 164, Verseifungszahlen von etwa 135 bis 178 und Tropfpunkte im Bereich von etwa 46 bis 48°C auf.

Zwar sind Ethoxylierungsprodukte von Wollwachs und von bestimmten Wollwachs-Derivaten, wie molekulardestillierten Wollwachsalkoholen und Wollwachssäuren, die bis zu 75 Mole Ethylenoxid pro Mol des Wollwachs oder Wollwachs-Derivats enthalten können, bereits bekannt und werden teilweise in Form von Handelsprodukten vertrieben. Diese Ethoxylierungsprodukte, die unterschiedlich wasserlöslich sind (teilweise nur in Wasser dispergierbar), werden vorzugsweise als Lösungsvermittler, Stabilisator, Gelbildner sowie als Hilfsemulgator in Verbindung mit anderen Emulgatoren zur Herstellung von W/O- und O/W-Emulsionen eingesetzt. Wo in Einzelfällen bei diesen bekannten Produkten in Abhängigkeit von der Länge des Polyoxyethylen-Restes der Einsatz als O/W-Emulgator vorgeschlagen wird, so erfolgt dies nahezu stets in Verbindung mit zumindest einer geringen Menge an Wollwachs, Wollwachsalkoholen oder anderen Emulgatoren, Cetylakohol oder Cetostearylakohol. Versuche haben ergeben, daß unter Verwendung dieser bekannten Ethoxylierungsprodukte als alleinigem O/W-Emulgator in den üblichen Mengen und unter den Bedingungen der Praxis keine über längere Zeit stabilen O/W-Emulsionen hergestellt werden konnten.

Im Gegensatz dazu sind die erfindungsgemäßen Ethoxylierungsprodukte, die durch Umsetzung von einem Mol der Ester aus Wollwachssäuren und Glycerin, insbesondere der Partialester, mit 0,5 bis 30 Mol Ethylenoxid erhältlich sind und die als feste, wachsartige Substanzen von gelblichweißer bis gelbbrauner Farbe anfallen, sämtlich in Wasser löslich und stellen ausgezeichnete O/W-Emulgatoren dar, die allein eingesetzt zur Herstellung stabiler O/W-Emulsionen hervorragend geeignet sind.

Es konnte nicht vorhergesehen werden und war daher überraschend, daß gemäß der Erfindung ausgehend von einem Produkt mit ausgesprochenen W/O-Emulgatoreigenschaften schon bei Anlagerung von nur sehr wenigen Ethylenoxid-Einheiten (Ester/Ethylenoxid-Molverhältnis beispielsweise: 1 : 2,9) ein guter Emulgator vom umgekehrten Typ (O/W-Emulgator) erhalten werden kann. Im Hinblick auf die bekannten Ethoxylierungsprodukte von Wollwachs-Derivaten, bei denen eine O/W-Emulgatorfunktion erst bei einer wesentlich höheren Zahl von Ethylenoxid-Einheiten im Molekül auftritt bzw. auftreten soll, war dies keineswegs zu erwarten.

Die Erfindung betrifft weiterhin ein Verfahren zur Herstellung der neuen O/W-Emulgatoren gemäß Anspruch 1, das dadurch gekennzeichnet ist, daß man in an sich bekannter Weise, vorzugsweise in Gegenwart eines alkalischen Katalysators (vgl. M.J. Schick: "Nonionic Surfactants", New York (1967) und N. Schönfeldt: "Grenzflächenaktive Äthylenoxid-Addukte", Suttgart (1976)), Wollwachssäuren-Glycerin-Ester, insbesondere die Glycerin-Partialester, im Temperaturbereich von etwa 120 bis 200°C mit Ethylenoxid umsetzt.

Man geht dabei so vor, daß in einem Druckgefäß die Wollwachssäuren-Glycerin-Ester vorgelegt werden und eine geringe Menge eines alkalischen Katalysators, wie z. B. Natrium- oder Kaliumhydroxid oder Natrium-oder Kaliummethylat, zugegeben und dann die Mischung unter Ausschluß von Wasser mit Ethylenoxid umgesetzt wird. Im allgemeinen werden hierbei Temperaturen im Bereich von etwa 120 bis 200°C angewandt und die Zugabe von Ethylenoxid wird so bemessen, daß der genannte Temperaturbereich weder unter- noch überschritten wird. Vorzugsweise wird die Umsetzung im unteren Teil des genannten Temperaturbereichs, bei etwa 120 bis 160°C, vorgenommen, um stärkere Verfärbungen der Reaktionsprodukte zu vermeiden. Zweckmäßig arbeitet man bei erhöhtem Druck in einer Inertgasatmosphäre, z. B. unter Verwendung von Stickstoff. Erforderlichenfalls kann das jeweilig erhaltene Umsetzungsprodukt mit Wasserstoffperoxid gebleicht werden.

Die Herstellung der als Ausgangsprodukt für die Ethoxylierung eingesetzten Wollwachssäuren-Glycerin-Ester ist aus der deutschen Patentschrift 2 023 786 und der deutschen Auslegeschrift 1 955 763 bekannt.

Sie kann beispielsweise aus Wollwachssäuren und Glycerin nach den üblichen, unter Wasserabspaltung verlaufenden Veresterungsverfahren erfolgen, in der Regel bei erhöhter Temperatur (bis etwa 230°C), wobei man die Kondensation durch Zusatz wasserbindender Mittel oder durch geeignete Katalysatoren sowie durch Anlegen eines milden Vakuums fördern kann. Durch entsprechende Reaktionsführung in Verbindung mit einer entsprechenden Abstimmung der Mengenverhältnisse der Reaktionspartner hat man es in der Hand, in welchem Umfang die OH-Gruppen der Ausgangssubstanzen umgesetzt werden (Möglichkeit der Bildung von Partialestern). Den Veresterungsgrad kann man an der Menge des abgeschiedenen Wassers kontrollieren oder aber durch Bestimmung der Säure- und Hydroxylzahl der Reaktionslösung.

Eine weitere Möglichkeit zur Herstellung der Wollwachssäuren-Glycerin-Ester besteht in der Umesterung von z. B. Wollwachssäuren-isopropylester mit Glycerin, die zweckmäßig im Temperaturbereich von etwa 100 bis 120°C in Anwesenheit einer geringen Menge eines alkalischen Katalysators vorgenommen werden kann.

Besonders gute Ausbeuten an Wollwachssäuren-Glycerin-Ester oder -Partialester in Form von sehr reinen, hellfarbenen Produkten werden dann erhalten, wenn man die Ester in der Weise herstellt, daß man das Gemisch aus Wollwachssäuren und Glycerin - ohne Zusatz eines Veresterungskatalysators oder eines Schleppmittels - unter Durchleiten von Wasserstoff und in Gegenwart von 0,01 bis 5 % eines Hydrierkatalysators (z. B. feinverteiltes Nickel oder auf Aktivkohle niedergeschlagenes Palladium oder Platin), bezogen auf das Gesamtgewicht der Reaktionspartner, so lange auf eine Temperatur von 200 bis 250°C erhitzt, bis die theoretisch errechnete Menge Wasser abgeschieden ist (DE-AS-1 955 763).

Das Verfahren zur Herstellung der erfindungsgemäßen neuen O/W-Emulgatoren wird anhand der folgenden Beispiele näher erläutert:

## Beispiel 1

In einem auf 6 bar ausgelegten 1-Liter-Glasautoklaven, der mit einem explosionsgeschützten Rührer, einer Heizung und einer Kühleinrichtung versehen war, wurden 145 g (0,35 Mol) eines Wollwachssäuren-Glycerin-Partialesters (VZ 137) und 2,5 g (45 mMol) Kaliumhydroxid-Pulver miteinander vermischt. Der Autoklav wurde dreimal mit Stickstoff gefüllt und wieder evakuiert. Danach wurde das Gemisch im Wasserstrahlvakuum eine Stunde lang bei 90°C getrocknet. Anschließend wurde Stickstoff bis zu einem Druck von 0,5 bar eingedrückt und der Autoklaveninhalt auf 130°C erwärmt.

Aus einer Vorlage wurden 462 g (10,5 Mol) Ethylenoxid mit Hilfe von Stickstoff mit einer solchen Geschwindigkeit in den Autoklaven gedrückt, daß eine Reaktionstemperatur von 150°C und ein Maximaldruck von 5 bar nicht überschritten wurde. Nach 150 Minuten wurde das letzte Ethylenoxid in den Autoklaven gedrückt. Nach weiteren 5 Minuten war das Ende der Reaktion am Druck- und Temperaturabfall zu erkennen. Sodann wurde das Reaktionsprodukt auf 90°C abgekühlt und im Wasserstrahlvakuum während 30 Minuten entgast. Nach Zutropfen von 5,9 g 30 %-igem Wasserstoffperoxid (1 %, bezogen auf die Produktmenge) und 30 Minuten Nachrühren bei 90°C wurde ein gelb gefärbtes Produkt erhalten, dessen OH-Zahl 76 betrug und dessen Verseifungszahl bei 28 lag.

**Beispiele 2 - 8**

Nach der im Beispiel 1 beschriebenen Arbeitsweise wurden die Wollwachssäuren-Glycerin-Partialester mit unterschiedlichen Mengen Ethylenoxid umgesetzt.

Die jeweiligen Versuchsbedingungen, Reaktionszeiten und Versuchsergebnisse sind zusammen mit den entsprechenden Daten (OH-Zahl = OHZ; Verseifungszahl = VZ) aus Beispiel 1 in der nachfolgenden Tabelle aufgeführt:

**Tabelle**

| Beispiel | Einsatz Ester (g) | Einsatz EO (g) | Ester/EO Molverhältnis | KOH (g) | Reaktions-temp.°C | Reaktions-zeit | OHZ End-pro-dukt | VZ End-pro-dukt |
|---|---|---|---|---|---|---|---|---|
| 1 | 145 | 462 | 1 : 30 | 2,5 | 130° - 150° | 14 Std. | 76 | 28 |
| 2 | 193 | 415 | 1 : 20 | 2,5 | 130° | 13 Std. | 96 | 37 |
| 3 | 209 | 326 | 1 : 14,5 | 2,5 | 140° | 10 Std. | 117 | 49 |
| 4 | 209 | 218 | 1 : 9,7 | 2,2 | 130° | 8 Std. | 141 | 62 |
| 5 | 360 | 194 | 1 : 5 | 2,2 | 135° - 140° | 7 Std. | 177 | 82 |
| 6 | 314 | 98 | 1 : 2,9 | 2,0 | 135° | 5 Std. | 202 | 100 |
| 7 | 418 | 90 | 1 : 2 | 2,0 | 135° | 4 Std. | 213 | 108 |
| 8 | 418 | 23 | 1 : 0,5 | 2,0 | 135° | 2 1/2 Std. | 221 | 129 |

EO = Ethylenoxid

Die Erfindung betrifft ferner kosmetische Mittel, die neben den üblichen Bestandteilen 1 bis 15 Gew.-%, vorzugsweise 2 bis 6 Gew.-%, bezogen auf die gesamte Zubereitung, mindestens einer Verbindung gemäß Anspruch 1 enthalten.

Die kosmetischen Mittel gemäß der Erfindung können in verschiedenen Formen vorliegen, insbesondere in Form von Cremes, Milch und Lotionen. Sie können in Anpassung an den jeweiligen Verwendungszweck in derartigen Mitteln übliche Komponenten in ebenfalls üblichen Mengenverhältnissen enthalten, wie Fettalkohole mit 12 bis 18 C-Atomen (z. B. Cetylalkohol, Myristylalkohol, Cetostearylalkohol), mittelkettige Triglyceride, esterartige Verbindungen (z. B. Isopropylmyristat, Isopropylpalmitat und Propylenglykolmonomyristat), 1,2-Propylenglykol, Mineralöl sowie Triäthanolamin (zur Neutralisation von Carboxylgruppen). Dabei hat es sich als zweckmäßig erwiesen, die Menge an Fettalkoholen in der kosmetischen Zubereitung auf maximal 6 Gew-%, bezogen auf die Gesamt-Zusammensetzung, zu begrenzen und die Menge an 1,2-Propylenglykol auf 5 Gew.-%. Daneben können die kosmetischen Mittel 0,01 bis 0,05 Gew.-%, bezogen auf die Gesamt-Zusammensetzung, an Antioxidantien, wie z. B. Butylhydroxytoluol oder Butylhydroxyanisol, Konservierungsmittel, wie Benzoesäurederivate, sowie Parfüm und gegebenenfalls 1 bis 4 Gew.-% eines Lichtschutzmittels, wie 2-Phenylbenzimidazol-5-sulfonsäure oder dessen Salze, enthalten.

Die jeweils einzusetzende Menge an Konservierungsmittel und Parfüm kann in Abhängigkeit von der Art des jeweiligen Produktes vom Fachmann durch einfaches Ausprobieren leicht ermittelt werden.

Der Wassergehalt der kosmetischen Mittel gemäß der Erfindung, die beständige O/W-Emulsionen darstellen, kann 60 bis 90 %, vorzugsweise 80 bis 90 %, der Gesamt-Zusammensetzung betragen.

Die Herstellung der kosmetischen Präparate erfolgt in üblicher Weise, indem die die Fettphase bildenden Bestandteile und die die Wasserphase bildenden Bestandteile unabhängig voneinander vermischt und getrennt auf etwa 75°C erwärmt werden. Anschließend wird bei dieser Temperatur die Wasserphase in die als Schmelze vorliegende Fettphase eingerührt, die so erhaltene Mischung (Emulsion) kaltgerührt, bei etwa 35° parfümiert und bei 32°C einmal auf Stufe 1 in einem üblichen Homogenisator homogenisiert. Dabei wird der Emulgator (polyethoxylierter Wollwachssäuren-Glycerin-Ester) zweckmäßig als Bestandteil der Fettphase behandelt und zusammen mit den übrigen Bestandteilen der Fettphase vermischt und erwärmt.

Die Viskosität der O/W-Emulsionen kann sowohl durch die Konsistenz der verwendeten Fettphase als auch durch die jeweils eingesetzte Wassermenge beeinflußt werden.

Die folgenden Beispiele 9 bis 11 sollen die Erfindung näher erläutern, ohne diese darauf zu beschränken, wobei jeweils die Bestandteile der Fettphase mit dem Buchstaben F und die der Wasserphase mit dem Buchstaben W gekenzeichnet wurden.

**Beispiel 9**

(O/W-Sonnenmilch)

| | |
|---|---|
| Emulgator (Prod., das pro Mol Wollwachssäuren-Glycerin-Ester 14,5 Mol EO enthält) | 3,00 g (F) |
| Cetylalkohol | 1,00 g (F) |
| Propylenglykolmonomyristat | 2,00 g (F) |
| Schaumverhütungsmittel ("Antischaum SH", Wacker: 0,04 g gelöst in 0,16 g Isopropylmyristat) | 0,20 g (F) |
| Antioxidans (Butylhydroxytoluol) | 0,03 g (F) |
| Triäthanolamin, rein | 0,20 g (F) |
| 2-Äthyl-hexyl-p-methoxizimtsäureester | 2,00 g (F) |
| Isopropylpalmitat | 4,50 g (F) |
| Glycerin | 2,50 g (W) |
| 2-Phenylbenzimidazol-5-sulfonsäure(Na-salz) ("Eusolex 232", Goodrich) | 1,00 g (W) |
| Acrylsäurepolymerisat ("Carbopol 940", Goodrich) | 0,15 g (W) |
| Wasser, vollst. entsalzt | 83,42 g (W) |
| Konservierungsmittel | q.s. |
| Parfum | q.s. |
| | 100,00 g |

**Beispiel 10**

(O/W-Creme)

| | |
|---|---|
| Emulgator (Prod., das pro Mol Wollwachs-säuren-Glycerin-Ester 2,9 Mol EO enthält) | 3,00 g (F) |
| Cetylstearylalkohol | 6,00 g (F) |
| Mineralöl (3°E/50°C) | 6,50 g (F) |
| Propylenglykolmonomyristat | 2,00 g (F) |
| Schaumverhütungsmittel ("Antischaum SH20", gebildet aus "Silikon-Antischaum SH", Wacker: 0,04 g + 0,16 g Isopropylmyristat) | 0,20 g (F) |
| Antioxidans (Butylhydroxytoluol) | 0,03 g (F) |
| Triathänolamin, rein | 0,30 g (F) |
| 1,2 - Propylenglykol | 2,50 g (W) |
| Acrylsäurepolymerisat ("Carbopol 934", Goodrich) | 0,30 g (W) |
| Wasser, vollstdg. entsalz | 79,17 g (W) |
| Konservierungsmittel | q.s. |
| Parfüm | q.s. |
| | 100,00 g |

6

**Beispiel 11**

(O/W-Lotion)

| | |
|---|---|
| Emulgator (Prod., das pro Mol Wollwachs-säuren-Glycerin-Ester 9,7 Mol EO enthält) | 3,00 g (F) |
| Cetylalkohol | 1,00 g (F) |
| Capryl-Caprinsäure-Triglycerid ("Miglyol 812", Dynamit Nobel AG) | 6,50 g (F) |
| Propylenglykolmonomyristat | 2,00 g (F) |
| Schaumverhütungsmittel aus: "Silikon-Antischaum SH": 0,04 g + Isopropylmyristat: 0,16 g | 0,20 g (F) |
| Antioxydans (Butylhydroxytoluol) | 0,03 g (F) |
| Triäthanolamin, rein | 0,15 g (F) |
| Glycerin | 2,50 g (W) |
| Acrylsäurepolymerisat ("Carbopol 934", Goodrich) | 0,15 g (W) |
| Wasser, vollst. entsalzt | 84,47 g (W) |
| Konservierungsmittel | q.s. |
| Parfüm | q.s. |
| | 100,00 g |

Gemäß den vorstehenden Beispielen 9 bis 11 werden in allen Fällen O/W-Emulsionen hoher Qualität erhalten, die selbst bei längerer Lagerung unter ungünstigen Bedingungen eine gute Stabilität zeigen und eine gute Hautund Schleimhautverträglichkeit sowie eine geringe Elektrolytempfindlichkeit aufweisen.

Die erfindungsgemäßen neuen Verbindungen sind daher wertvolle O/W-Emulgatoren mit ausgezeichneten rückfettenden Eigenschaften, die neben ihrer sehr guten emulgierenden Wirkung gute Lösungsvermittler für ätherische Öle, Riechstoffe, Vitaminöle sowie für eine große Anzahl kosmetischer und pharmazeutischer Wirkstoffe sind.

**Patentansprüche**

1. Neue Öl-in-Wasser-(O/W)-Emulgatoren für kosmetische Zwecke in Form von polyethoxylierten Wollwachs-Derivaten erhältlich durch Umsetzung der Voll- oder Partialester aus Wollwachssäuren und Glycerin mit Ethylenoxid, wobei das Molverhältnis von Ester zu Ethylenoxid 1: (0,5 bis 30) beträgt.

2. Verfahren zur Herstellung von Verbindungen gemäß Anspruch 1, dadurch gekennzeichnet, daß man in an sich bekannter Weise, vorzugsweise in Gegenwart eines alkalischen Katalysators, die Wollwachssäuren-Glycerin-Ester, insbesondere die Glycerin-Partialester, im Temperaturbereich von etwa 120 bis 200°C, zweckmäßig bei erhöhtem Druck und in einer Inertgasatmosphäre, mit Ethylenoxid umsetzt.

3. Kosmetische Mittel, dadurch gekennzeichnet, daß sie neben den üblichen Bestandteilen 1 bis 15 Gew.-% vorzugsweise 2 bis 6 Gew.-%, bezogen auf die gesamte Zubereitung, mindestens einer Verbindung gemäß Anspruch 1 enthalten.

## Claims

1. Novel oil-in-water (O/W) emulsifiers for cosmetic purposes in the form of polyethoxylated wool wax derivatives, obtainable by reaction of full or partial glycerol esters of wool wax acids with ethylene oxide, the ester: ethylene oxide molar ratio being 1 : (0.5 to 30).

2. Process for the preparation of compounds according to Claim 1, characterized in that the glycerol esters of wool wax acids, in particular the partial glycerol esters, are reacted with ethylene oxide in a fashion which is known per se, preferably in the presence of an alkaline catalyst, in a temperature range from about 120 to 200°C, expediently at increased pressure and in an inert gas atmosphere.

3. Cosmetics, characterized in that they contain, in addition to the conventional components, 1 to 15 % by weight, preferably 2 to 6 % by weight, relative to the total preparation, of at least one compound according to Claim 1.

## Revendications

1. Nouveaux émulsifiants huile-dans-eau (H-E) pour usages cosmétiques sous la forme de dérivés polyéthoxylés de la lanoline pouvant s'obtenir par réaction des esters complets ou partiels des acides de lanoline et de glycérine avec l'oxyde d'éthylène, le rapport molaire des esters à l'oxyde d'éthylène étant de 1 : (0,5 à 30).

2. Procédé de préparation de composés suivant la revendication 1, caractérisé en ce qu'on fait réagir d'une façon connue, de préférence en présence d'un catalyseur alcalin, les esters d'acides de lanoline et de glycérine, en particulier les esters partiels de glycérine, dans l'intervalle de température d'environ 120 à 200°C, avantageusement sous pression élevée et sous atmosphère inerte, avec l'oxyde d'éthylène.

3. Produits cosmétiques, caractérisés en ce qu'ils contiennent, en plus des constituants habituels, 1 à 15 % en poids et de préférence 2 à 6 % en poids, sur base de la préparatiom complète, d'au moins un composé suivant la revendication 1.